(19) **Europäisches Patentamt**
**European Patent Office**
**Office européen des brevets**

(11) **EP 2 189 184 B1**

(12) **EUROPÄISCHE PATENTSCHRIFT**

(45) Veröffentlichungstag und Bekanntmachung des Hinweises auf die Patenterteilung:
**17.11.2010 Patentblatt 2010/46**

(51) Int Cl.:
*A61N 5/10* (2006.01)   *G21K 1/10* (2006.01)

(21) Anmeldenummer: **09169731.8**

(22) Anmeldetag: **08.09.2009**

(54) **Vorrichtung und Verfahren zur Reduzierung der Strahlaufweitung einer Strahlung**

Device and method for reducing the beam dispersion of a beam

Dispositif et procédé de réduction de l'élargissement de rayon d'un rayonnement

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **20.11.2008 DE 102008058299**

(43) Veröffentlichungstag der Anmeldung:
**26.05.2010 Patentblatt 2010/21**

(73) Patentinhaber: **SIEMENS AKTIENGESELLSCHAFT**
**80333 München (DE)**

(72) Erfinder: **Mattern, Detlef**
**91056 Erlangen (DE)**

(56) Entgegenhaltungen:
**WO-A2-02/07817**

• **KARLSSON MAGNUS G ET AL: "Treatment head design for multileaf collimated high-energy electrons" MEDICAL PHYSICS, AIP, MELVILLE, NY, US, Bd. 26, Nr. 10, 1. Oktober 1999 (1999-10-01), Seiten 2161-2167, XP012010610 ISSN: 0094-2405**

• **SAFAI SAIROS ET AL: "Comparison between the lateral penumbra of a collimated double-scattered beam and uncollimated scanning beam in proton radiotherapy" PHYSICS IN MEDICINE AND BIOLOGY, TAYLOR AND FRANCIS LTD. LONDON, GB, Bd. 53, Nr. 6, 21. März 2008 (2008-03-21), Seiten 1729-1750, XP007912126 ISSN: 0031-9155**

• **KARLSSON MAGNUS G ET AL: "Electron beam collimation with focused and curved leaf end MLCs-Experimental verification of Monte Carlo optimized designs" MEDICAL PHYSICS, AIP, MELVILLE, NY, US, Bd. 29, Nr. 4, 1. April 2002 (2002-04-01), Seiten 631-637, XP012011769 ISSN: 0094-2405**

**Beschreibung**

Die Erfindung betrifft eine im Patentanspruch 1 angegebene Strahlentherapieanlage und ein im Patentanspruch 4 angegebenes Verfahren zur Reduzierung einer Strahlaufweitung einer Strahlentherapieanlage.

Allgemein wird als Strahlentherapie ein medizinisches Therapieverfahren zur Behandlung von Tumorerkrankungen bezeichnet. Dabei wird hochenergetische Photonenstrahlung (Röntgenstrahlung, Gammastrahlung) oder Partikelstrahlung (Elektronen, Protonen, Ionen) auf einen zu behandelnden Körperbereich eines Patienten gelenkt. Eine Strahlenapplizierung kann aber auch in nicht-therapeutischen Bereichen eingesetzt werden, wie beispielsweise bei der Bestrahlung von Phantomen oder nicht-lebenden Körpern im Rahmen von Forschungsarbeiten, oder bei der Bestrahlung von Materialen.

Für die Partikeltherapie wird hochenergetische Partikelstrahlung mit einer Beschleunigungsanlage erzeugt. Die auf hohe Energien beschleunigten Partikel werden zu einem Partikelstrahl geformt und anschließend auf das zu bestrahlende Gewebe gerichtet. Die Partikel dringen in das zu bestrahlende Gewebe ein und geben dort in einem umschriebenen Bereich ihre Energie ab. Die Eindringtiefe des Partikelstrahls in das zu bestrahlende Gewebe hängt vornehmlich von der Energie des Partikelstrahls ab. Je höher die Energie des Partikelstrahls, desto tiefer dringen die Partikel in das zu bestrahlende Gewebe ein. Im Vergleich zu herkömmlichen Bestrahlungsverfahren, die mit Röntgen- und Elektronenstrahlen arbeiten, zeichnet sich die Partikeltherapie dadurch aus, dass die Energie der Partikel in einem umschriebenen und abgrenzbaren Bereich abgegeben wird. Hierdurch kann eine Bestrahlung z.B. eines Tumors genauer erfolgen und umliegendes Gewebe kann besser geschont werden.

Die Partikeltherapie wird üblicherweise in einer speziellen Partikeltherapieanlage durchgeführt, in der einerseits in einem Bereich der Partikelstrahl erzeugt und in der Regel zu mehreren Bestrahlungsräumen geführt wird, und in der andererseits in einem anderen Bereich verschiedene Räume vorhanden sind, in denen Patienten für eine bevorstehende Bestrahlungssitzung vorbereitet oder während einer Bestrahlungssitzung bestrahlt werden.

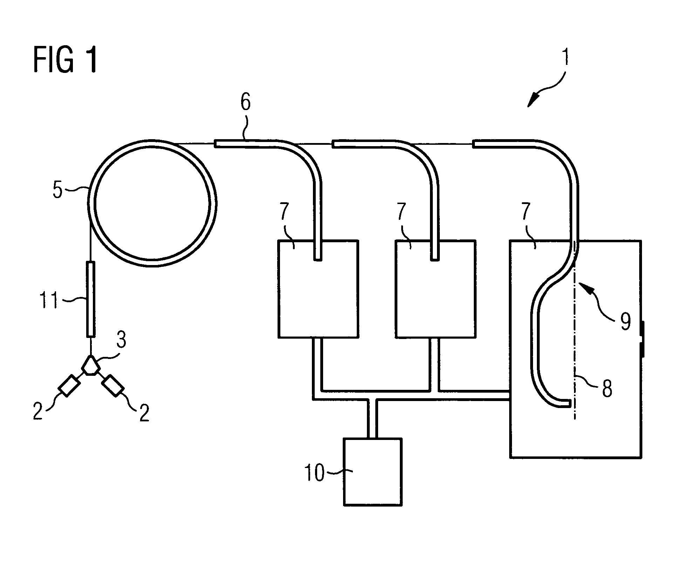

Figur 1 zeigt eine schematischen Übersicht eines Aufbaus einer Partikeltherapieanlage 1 gemäß der nachveröffentlichten DE 10 2008 005 068 A1. Als Partikel werden vornehmlich Ionen wie beispielsweise Protonen, Pionen, Heliumionen oder Kohlenstoffionen eingesetzt. Die Partikel werden in einer Partikelquelle 2 erzeugt. Wenn, wie in Figur 1 dargestellt, zwei Partikelquellen 2 vorhanden sind, die zwei verschiedene Ionenarten erzeugen, kann zwischen diesen beiden Ionenarten innerhalb kürzester Zeit umgeschaltet werden. Dazu wird beispielsweise ein Schaltmagnet 3 verwendet, der zwischen den Ionenquellen 2 einerseits und einem Vorbeschleuniger 4 andererseits angeordnet ist. Dadurch kann die Partikeltherapieanlage 1 mit Protonen und mit Kohlenstoffionen gleichzeitig betrieben werden.

Die von der oder einer der Ionenquellen 2 erzeugten und gegebenenfalls mit dem Schaltmagneten 3 ausgewählten Ionen werden in dem Vorbeschleuniger 4 auf ein erstes Energieniveau beschleunigt. Der Vorbeschleuniger 4 ist beispielsweise ein Linearbeschleuniger. Anschließend werden die Partikel in einen Beschleuniger 5, beispielsweise ein Synchrotron oder Zyklotron, eingespeist. In dem Beschleuniger 5 werden sie auf hohe Energien, wie sie zur Bestrahlung nötig sind, beschleunigt. Nachdem die Partikel den Beschleuniger 5 verlassen haben, führt ein Hochenergiestrahl-Transportsystem 6 den Partikelstrahl zu einem oder mehreren Bestrahlungsräumen 7. In einem Bestrahlungsraum 7 werden die beschleunigten Partikel auf einen zu bestrahlenden Körperteil gerichtet. Je nach Ausgestaltung erfolgt dies von einer festen Richtung aus oder aber über eine um eine Achse 8 bewegliche rotierbare Gantry 9 von verschiedenen Richtungen aus.

Weiterhin weist die Partikeltherapieanlage 1 verschiedene weitere Räume 10 auf, in denen beispielsweise Patienten auf eine bevorstehende Bestrahlungssitzung bzw. für eine bevorstehende Untersuchung vorbereitet werden. Diese weiteren Räume 10 sowie die Bestrahlungsräumen 7 stehen untereinander über Korridore in Verbindung.

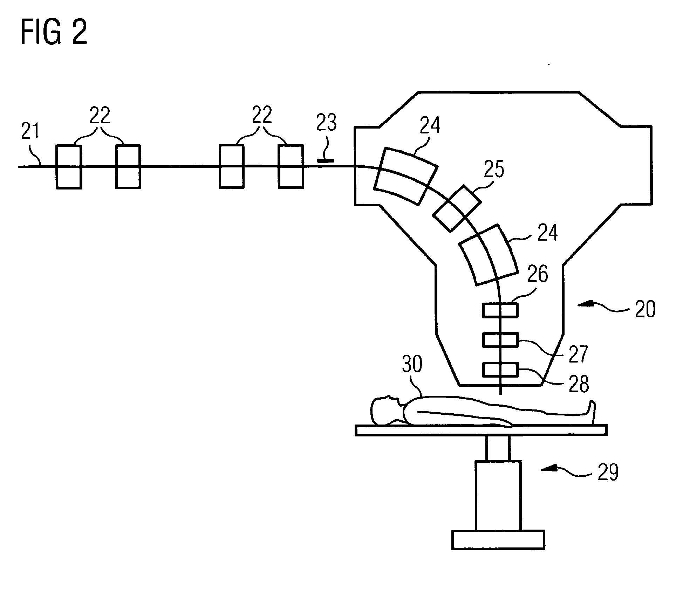

Figur 2 zeigt beispielhaft eine schematische Übersicht des "Innenlebens" einer Protonenbestrahlungsvorrichtung gemäß EP 0 864 337 A2. Nach einer Erzeugung und Beschleunigung eines Protonenstrahls 21 wird sein Querschnitt durch Magnetsysteme 22 größenmäßig angepasst. Durch eine Schalteinheit 23 wird sichergestellt, dass der Protonenstrahl 21 jederzeit abgeschaltet werden kann. Anschließend gelangt der Protonenstrahl 21 in einen Strahlkopf 20, auch "Nozzle" genant. Der Strahlkopf 20 umfasst zwei Umlenkmagnete 24, einen den Protonenstrahl 21 fokussierenden Quadrupolmagneten 25, ein die Energie des Partikelstrahls 21 justierendes Einstellsystem 26, eine Kollimatoreinheit 27 zur Anpassung der Strahlform sowie ein Detektorsystem 28 zur Überwachung der abgegebenen Strahldosis. Anschließend verlässt der Protonenstrahl 21 den Strahlkopf 20 und trifft auf einen auf einem Behandlungstisch 29 immobilisierten Patienten 30.

Bei der Planung von medizinischen Bestrahlungen ist es äußerst wichtig, die zwischen Strahlaustritt und Patient in Luft zurückgelegte Strecke zu berücksichtigen, da es dabei zu einer Strahlaufweitung infolge der sogenannten Vielfachstreuung kommt.

Es ist Aufgabe der Erfindung eine Vorrichtung und ein dazugehöriges Verfahren anzugeben, mit denen flexibler und unabhängiger von einer Luftstrecke geplant werden kann.

Gemäß der Erfindung wird die gestellte Aufgabe mit der Strahlentherapieanlage des unabhängigen Patentanspruchs 1 und dem Verfahren zur Reduzierung einer Strahlaufweitung einer Strahlentherapieanlage des unabhängigen Patentanspruchs 4. Aus Karlsson et al, med Phys. 26(10), Oktober 1999, S.2161-2167 ist eine Erfindung Vorrichtung zur Verringerung der Strahlaufweitung einer Strahlung, bekannt, wobei zwischen einem Strahlkopf und einem zu bestrahlendem Objekt ein Mittel angeordnet ist, das mit einem Gas oder einem Gasgemisch gefüllt ist, dessen mittlere Kernladungszahl kleiner als die von Luft ist, und das in seiner räumlichen Ausdehnung veränderbar ist. Da im Gas die Vielfachstreuung geringer ist, wird der Strahl vorteilhaft weniger stark aufgeweitet.

Erfindungsgemäss ist das Mittel einen in der Richtung zum Objekt seine Länge veränderbarer Faltenbalg. Dadurch kann das Mittel auf einen variablen Abstand zu einem Objekt angepasst werden.

Das Gas kann Helium sein. Dies bietet den Vorteil, dass Helium preiswert ist.

In einer Weiterbildung können Kaptonfolien die beiden Stirnseiten des Mittels, d.h. die Seiten für den Strahlein- und austritt, bilden. Die Kaptonfolien haben keinen störenden Einfluss auf die Strahlung.

Erfindungsgemäss umfasst die Vorrichtung einen Strahlkopf, an dem das Mittel angeordnet ist. An einem Strahlkopf kann mit Hilfe einer Verschiebevorrichtung das Mittel sicher und einfach bedienbar montiert werden.

Die Erfindung beansprucht auch ein Verfahren zur Reduzierung der Strahlaufweitung einer Strahlung, wobei bei einer Strahlenäpplizierung in nicht therapeutischen Bereichen, in Strahlrichtung nach einem Strahlkopf der Strahlentherapieanlange ein Faltenbalg angeordnet wird, der mit einem Gas oder einem Gasgemisch gefüllt wird, dessen mittlere Kernladungszahl kleiner als die von Luft ist, wobei die Länge des Faltenbalgs in Strahlrichtung veränderbar ist.

Die Strahlung kann Partikelstrahlung oder Röntgenstrahlung sein.

Weitere Besonderheiten und Vorteile der Erfindung werden aus den nachfolgenden Erläuterungen mehrerer Ausführungsbeispiele anhand von schematischen Zeichnungen ersichtlich.

Es zeigen:

Figur 1: eine Übersichtsansicht einer Strahlentherapieanla- ge gemäß Stand der Technik,
Figur 2: eine Detailansicht einer Partikeltherapieanlage gemäß Stand der Technik,
Figur 3: eine Schnittansicht eines Teils eines Strahlkopfs mit Bestrahlungsobjekt,
Figur 4: eine Schnittansicht eines Teils eines Strahlkopfs mit ausgezogenem Faltenbalg,
Figur 5: eine Schnittansicht eines Teils eines Strahlkopfs mit eingefahrenem Faltenbalg,
Figur 6: ein Diagramm des mittleren Streuwinkels für Luft und Helium und
Figur 7: ein Diagramm des Quotienten der mittleren Streu- winkel für Luft und Helium.

Die folgenden Figuren zeigen die erfindungsgemäße Vorrichtung in einer Partikeltherapieanlage. Ebenso kann die Erfindung auch in anderen medizinischen und nicht-medizinischen Bestrahlungsanlagen zum Einsatz kommen.

Bei der Tumortherapie mit ionisierender Strahlung, insbesondere Partikelstrahlung wie Protonen und Elektronen, werden die Partikel im Hochvakuum erzeugt und auf hohe Energien beschleunigt. Damit die Strahlung in einem Tumorvolumen im Körper eines Patienten seine Wirkung entfalten kann, muss sie einen Beschleunigerapparat durch ein möglichst dünnes Vakuumfenster verlassen, eine Strecke mit typischerweise 1 Meter in Luft zurücklegen, ehe die Strahlung wie geplant in den Patienten eindringen kann.

Die Luftstrecke ist in der Regel durch die Art der Patientenlagerung und die Fokussierung der Strahlung vorgegeben und daher nicht beliebig veränderbar. Die gesamte Behandlungsplanung basiert auf der unveränderbaren Lage eines sogenannten Isozentrums. Der Patient wird auf einer Patientenlagerungsvorrichtung so positioniert, dass sich das Isozentrum im Tumorvolumen befindet.

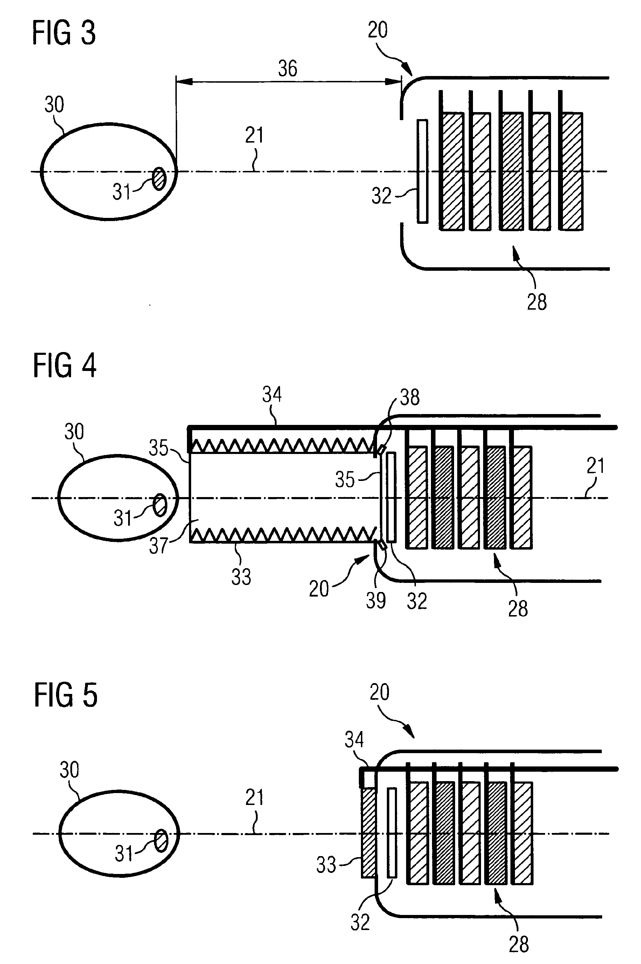

Figur 3 zeigt den oben beschriebenen Zusammenhang anhand einer Übersichtsdarstellung. Dargestellt ist der vordere Teil eines Strahlkopfs 20 einer Partikeltherapieanlage. Zu sehen sind in seinem Inneren ein aus mehreren Detektoren gebildetes Detektorsystem 28 und eine Filtereinheit 32, durch welche ein Partikelstrahl 21, beispielsweise eine Protonenstrahl, hindurch tritt. Nach Verlassen des Strahlkopfs 20 passiert der Partikelstrahl 21 eine Luftstrecke 36, ehe er in einen Patienten 30 eindringt und dort auf einen Tumor 31 trifft. Im Tumor 31 gibt der Partikelstrahl 21 seine Energie ab und kann so Krebszellen schädigen bzw. zerstören. Infolge der sogenannten Vielfachstreuung kommt es zu einer Strahlweitung des Partikelstrahls, die bei einer Behandlungsplanung berücksichtigt werden muss.

Wenn geladene Teilchen ein Medium durchsetzen, werden sie am Coulomb-Potential der Kerne und Elektronen gestreut. Es kommt dabei zu vielen Streuakten mit geringfügigen Ablenkungen. Die Streuwinkelverteilung aufgrund der Coulomb-Streuung wird durch die Moliere-Theorie beschrieben. Aus "Teilchendetektoren / Claus Gruppen, BI-Wissenschaftsverlag, 1993" ergibt sich der mittlere Streuwinkel Θ zu

$$\theta = \frac{13.6 MeV}{\beta cp} z \sqrt{\frac{x}{X_0}} \left[ 1 + 0.038 \ln\left( \frac{x}{X_0} \right) \right], \tag{1}$$

wobei p den Impuls in MeV/c, βc die Geschwindigkeit und z die Ladung des gestreuten Teilchens darstellt. $x/X_0$ ist die Dicke des streuenden Mediums in Einheiten der Strahlungslänge

$$X_0 = \frac{A}{4\alpha N_A Z^2 r_e^2 \ln\left(183 \cdot Z^{-\frac{1}{3}}\right)},$$ (2)

wobei Z und A die Ladungszahl, bzw. das Atomgewicht des absorbierenden Mediums, $\alpha$ die Feinstrukturkonstante, $r_e$ den klassischen Elektronenradius und $N_A$ die Avogadro-Konstante darstellen.

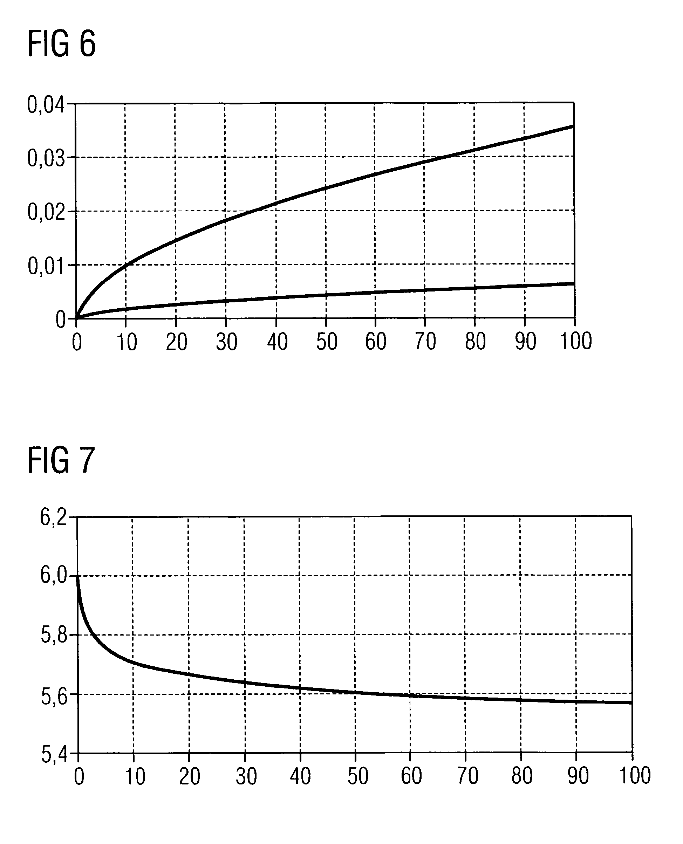

Setzt man in die Gleichungen (1) und (2) die Materialdaten für Luft ein, ergibt sich für Protonen mit einer Energie von 50MeV eine Abhängigkeit des mittleren Streuwinkels Θ in Rad als Funktion der in Luft zurückgelegten Strecke x in cm die Kurve A gemäß Figur 6. Die mittlere Aufweitung des Strahls ergibt sich für kleine Winkel näherungsweise durch Multiplikation des mittleren Streuwinkels Θ in Rad mit der Strecke x. Für 100 cm ergeben sich aus der Kurve A der Figur 6 etwa 35 mm.

Die erfinderische Idee besteht nun darin, die Luft zwischen dem Strahlkopf und dem Patienten durch ein Edelgas, beispielsweise Helium, oder ein Edelgasgemisch zumindest teilweise zu ersetzen, da für Elemente mit kleiner Kernladungszahl und kleiner Dichte der mittlere Streuwinkel kleiner ist. In einer erfindungsgemäßen Ausführungsform wird das Edelgas in einem Faltenbalg 33 gemäß der Figuren 4 und 5 gefasst.

In Figur 4 ist der vordere Teil eines Strahlkopfs 20 einer Partikeltherapieanlage dargestellt. Zu sehen sind in seinem Inneren ein aus mehreren Detektoren gebildetes Detektorsystem 28 und eine Filtereinheit 32, durch welche ein Partikelstrahl 21, beispielweise eine Protonenstrahl, hindurch tritt. Nach Verlassen des Strahlkopfs 20 durchwandert der Partikelstrahl 21 einen ausgezogenen Faltenbalg 33, ehe er in einen Patienten 30 eindringt und dort auf einen Tumor 31 trifft. Im Tumor 31 gibt der Partikelstrahl 21 seine Energie ab und kann so Krebszellen schädigen bzw. zerstören. Der Faltenbalg 33 ist mit Helium gefüllt und an seinen Stirnseiten durch Kaptonfolien 35 abgeschlossen. Durch ein Einlassventil 38 gelangt Helium in das Innere 37 des Faltenbalgs 33. Durch ein Auslassventil 39 kann Helium bei Bedarf aus dem Faltenbalg 33 entweichen. Nicht dargestellte Zu- und Ableitungen sowie Sensoren und eine Steuereinheit regeln die Zu- und Abfuhr des Heliums je nach Ausziehgröße des Faltenbalgs 33. Mit Hilfe einer Verstelleinheit 34 kann die erforderliche Länge des Faltenbalgs 33 eingestellt werden. Sie wird im Wesentlichen durch den Abstand Patient 30 zum Strahlaustritt aus dem Strahlkopf 20 bestimmt.

In Figur 5 ist der Faltenbalg 33 gemäß Figur 4 in einer eingefahrenen Position dargestellt. In Figur 5 ist der vordere Teil eines Strahlkopfs 20 einer Partikeltherapieanlage dargestellt. Zu sehen sind in seinem Inneren ein aus mehreren Detektoren gebildetes Detektorsystem 28 und eine Filtereinheit 32, durch welche ein Partikelstrahl 21, beispielweise eine Protonenstrahl, hindurch tritt. Nach Verlassen des Strahlkopfs 20 durchwandert der Partikelstrahl 21 den eingefahrenen Faltenbalg 33, ehe er in einen Patienten 30 eindringt und dort auf einen Tumor 31 trifft. Im Tumor 31 gibt der Partikelstrahl 21 seine Energie ab und kann so Krebszellen schädigen bzw. zerstören.

Mit Hilfe der Gleichungen (1) und (2) lässt sich für Helium der mittlere Streuwinkel Θ bestimmen. Die Kurve B in Figur 6 zeigt für Protonen mit einer Energie von 50MeV die Abhängigkeit des mittleren Streuwinkels Θ in Rad als Funktion der in Helium zurückgelegten Strecke x in cm. Die mittlere Aufweitung des Strahls ergibt sich für kleine Winkel näherungsweise durch Multiplikation des mittleren Streuwinkels Θ in Rad mit der Strecke x. Für 100 cm ergeben sich aus Kurve B der Figur 6 etwa 6 mm. Somit ergibt sich abhängig von der durchlaufenen Strecke x eine Reduktion des mittleren Streuwinkels Θ um etwa einen Faktor 5 bis 6. In Figur 7 ist dieses Verhältnis als Kurve C in Abhängigkeit der Länge x in cm dargestellt.

Analog kann auch die Strahlaufweitung von Photonenstrahlung, insbesondere von Röntgenstrahlung, reduziert werden.

Bezugszeichenliste

| 1 | Partikeltherapieanlage |
| 2 | Partikelquelle |
| 3 | Schaltmagnet |
| 4 | Vorbeschleuniger |
| 5 | Beschleuniger / Zyklotron |

6    Hochenergiestrahl-Transportsystem
7    Bestrahlungsraum
8    Achse
9    Gantry
10    weiterer Raum
20    Strahlkopf / Nozzle
21    Protonenstrahl / Partikelstrahl
22    Magnetsystem
23    Schalteinheit
24    Umlenkmagnet
25    Quadrupolmagnet
26    Einstellsystem
27    Kollimatoreinheit
28    Detektorsystem
29    Behandlungstisch
30    Patient
31    Tumor
32    Filtereinheit
33    Faltenbalg
34    Verstelleinheit
35    Kaptonfolie
36    Luftstrecke
37    Heliumfüllung
38    Einlassventil
39    Auslassventil

**Patentansprüche**

1.    Strahlentherapieanlage (1) mit einem Strahlkopf (20), **gekennzeichnet durch**:

   einen an dem Strahlkopf in Strahlrichtung (21) angeordneten Faltenbalg (33), der mit einem Gas (37) oder Gasgemisch gefüllt ist, dessen mittlere Kernladungszahl kleiner als die von Luft ist, wobei die Länge des Faltenbalgs in Strahlrichtung veränderbar ist.

2.    Anlage nach Anspruch 1,
      **dadurch gekennzeichnet,**
      **dass** das Gas (37) Helium ist.

3.    Anlage nach Anspruch 1 oder 2,
      **gekennzeichnet durch**:

   zwei Kaptonfolien (35), welche die beiden Stirnseiten des Faltenbalgs (33), d.h. die Seiten für einen Strahlein- und austritt, bilden.

4.    Verfahren zur Reduzierung einer Strahlaufweitung einer Strahlentherapieanlage (1),
      **dadurch gekennzeichnet, dass** bei einer Strahlenapplizierung in nicht-therapeutischen Bereichen, in Strahlrichtung (21) nach einem Strahlkopf (20) der Strahlentherapieanlage (1) ein Faltenbalg (33) angeordnet wird, der mit einem Gas (37) oder Gasgemisch gefüllt ist, dessen mittlere Kernladungszahl kleiner als die von Luft ist, wobei die Länge des Faltenbalgs in Strahlrichtung veränderbar ist.

**Claims**

1.    Radiation therapy system (1) comprising a beam head (20), **characterised by**:

   a bellows (33) arranged on the beam head in the beam direction (21), said bellows (33) being filled with a gas (37) or gas mixture, the average atomic number of which is smaller than that of air, with the length of the bellows being changeable in the beam direction.

2. System according to claim 1,
   **characterised in that**
   the gas (37) is helium.

3. System according to claim 1 or 2,
   **characterised by**:

   two Kapton films (35) that form the two front sides of the bellows (33) i.e. the sides for a beam input and output.

4. Method for reducing the widening of a beam in a radiation therapy system (1),
   **characterised in that**
   when applying radiation in non-therapeutic areas, a bellows (33) is arranged in the beam direction (12) downstream of a beam head (20) of the radiation therapy system (1), said bellows (33) being filled with a gas (37) or a gas mixture, the average atomic number of which is smaller than that of air, with the length of the bellows being changeable in the beam direction.

**Revendications**

1. Installation de radiothérapie (1) possédant une tête de rayonnement (20), **caractérisée par** : un soufflet (33) disposé sur la tête de rayonnement dans la direction de rayonnement (21), qui est rempli d'un gaz (37) ou d'un mélange de gaz, dont le numéro atomique moyen est inférieur à celui de l'air, la longueur du soufflet étant variable dans la direction de rayonnement.

2. Installation selon la revendication 1, **caractérisée en ce que** le gaz (37) est de l'hélium.

3. Installation selon la revendication 1 ou 2, **caractérisée par** deux feuilles de kapton (35) qui forment les deux côtés frontaux du soufflet (33), c'est-à-dire les côtés pour une entrée et une sortie du faisceau.

4. Procédé pour la réduction d'une dispersion de faisceau d'une installation de radiothérapie (1), **caractérisé en ce qu'**on dispose un soufflet (33), lors d'une application de faisceaux dans des domaines non thérapeutiques, dans la direction de rayonnement (21) après une tête de rayonnement (20) de l'installation de radiothérapie (1), qui est rempli d'un gaz (37) ou d'un mélange de gaz, dont le numéro atomique moyen est inférieur à celui de l'air, la longueur du soufflet étant variable dans la direction de rayonnement.

FIG 1

FIG 2

FIG 3

FIG 4

FIG 5

## FIG 6

## FIG 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008005068 A1

- EP 0864337 A2

**In der Beschreibung aufgeführte Nicht-Patentliteratur**

- **Karlsson et al.** *med Phys.,* Oktober 1999, vol. 26 (10), 2161-2167

- Teilchendetektoren / Claus Gruppen. BI-Wissenschaftsverlag, 1993